# EUROPEAN PATENT APPLICATION

(11) **EP 4 509 119 A1**
(43) Date of publication of application: **19.02.2025**
(21) Application number: 23788572.8
(22) Date of filing: 11.04.2023
(51) Int. Cl.: A61K 31/05, A61K 31/155, A61K 45/06, A61P 43/00, A61K 8/34, A61K 8/43, A61Q 19/08

(54) **COMPOSITION FOR REJUVENATION OF SENESCENT CELLS OR DELAY OR PREVENTION OF CELLULAR AGING**

(30) Priority: 15.04.2022 KR 20220047161
(71) Applicant: Lono Jaeyak, Inc., Gunsan-si Jeonbuk-do 54027 (KR)
(72) Inventor: SON, Seung Cheol, Incheon 22006 (KR); YANG, Eun Jae, Seoul 06929 (KR)
(74) Representative: Jacobi, Markus Alexander
(86) International application number: PCT/KR2023/004865
(87) International publication number: WO 2023/200223

(57) **Abstract**

A composition for the rejuvenation of senescent cells or the delay or prevention of cellular senescence, of the present invention, comprises: an ATM kinase inhibitor and a ROCK inhibitor; and at least one selected from the group consisting of a Bcl-2 inhibitor, a SIRT1 activator, and an AMPK activator.

## Description

### [Technical Field]

The present invention relates to a composition for rejuvenation of senescent cells or delay or prevention of cellular senescence. More specifically, the present invention relates to a composition capable of rejuvenating senescent cells or delaying or preventing cellular senescence through a reverse senescence effect that reverts senescent cells to a young cellular state, such as by causing senescent cells that have reached the Hayflick limit to resume cell division or by reducing the accumulation of undesirable metabolites due to cellular senescence, for example, lipofuscin.

### [Background Art]

As life expectancy increases due to the advancements in medical technology, problems related to aging-related diseases are increasing. Therefore, many researchers are trying to elucidate the mechanisms of aging and, based on this, to respond to aging-related problems, for example, age-related diseases.

Cellular senescence refers to a process in which the characteristics and functions of cells deteriorate and proliferation stops, and generally, it refers to the loss of ability to divide. Unlike cancer cells, normal cells reach the Hayflick limit after 40 to 60 cell divisions, at which point they can no longer divide. In addition, when cells age, unlike young cells, metabolic waste, such as lipofuscin, is accumulated, causing various degenerative diseases.

Various studies have been conducted to solve this problem of cellular senescence, and in the 2010s, through an experiment to remove only senescent cells from old mice using genetic modification technology, it was discovered that when senescent cells in an aged individual are removed, both the lifespan and healthy lifespan of the aged individual increase, thereby opening the door to the field of senotherapeutics. Since then, the development of senolytics, which are compounds that remove senescent cells, and senomorphics, compounds that suppress the characteristics of senescent cells, has been actively carried out.

Although it is true that anti-aging drugs increase lifespan and delay aging, the effects have not been so dramatic to date, and no senotherapeutic that helps senescent cells to overcome the Hayflick limit and divide as much as young cells has yet been reported.

Since the causes of aging are multifactorial, the cell signaling pathways involved are also diverse. Recently, it has been reported that when treated with an ataxia telangiectasia mutated (ATM) kinase inhibitor (ATM kinase inhibitor) and a Rho-associated, coiled-coil containing protein kinase (ROCK) inhibitor, cell division resumes as though the cells have been rejuvenated and become young cells. In particular, it has been recently reported that when simultaneously treated with an ATM kinase inhibitor KU60019 and a ROCK inhibitor Y27632, a synergistic effect is exhibited in this rejuvenation effect.

The inventors of the present invention sought to further develop these existing technologies to more effectively solve the problem of cellular senescence and further develop technologies capable of preventing, ameliorating, and treating cellular senescence-related diseases.

Related Art Document: (Non-Patent Document 1) Eun Jae Yang, "Synergistic revitalization of senescent cells via sequential activation of FOXM1 and E2F1," Daegu Gyeongbuk Institute of Science and Technology, Department of New Biology, doctoral dissertation, 2022

### [Disclosure]

### [Technical Problem]

The main object of the present invention is to effectively solve the problem of cellular senescence and to provide a composition capable of rejuvenating senescent cells or delaying or preventing cellular senescence through a reverse senescence effect that reverts senescent cells to a young cellular state, such as by causing senescent cells that have reached the Hayflick limit to resume cell division or by reducing the accumulation of undesirable metabolites due to cellular senescence.

### [Technical Solution]

The present invention provides a composition for rejuvenating senescent cells or delaying or preventing cellular senescence, including an ataxia telangiectasia mutated (ATM) kinase inhibitor and a Rho-associated, coiled-coil containing protein kinase (ROCK) inhibitor, and one or more selected from the group consisting of a B-cell lymphoma (Bcl)-2 inhibitor, a sirtuin 1 (SIRT1) activator, and an AMP-activated protein kinase (AMPK) activator.

### [Advantageous Effects]

The composition of the present invention can rejuvenate senescent cells or delay or prevent cellular senescence through a reverse senescence effect that reverts senescent cells to a young cellular state, such as by causing senescent cells that have reached the Hayflick limit to resume cell division or by reducing the accumulation of undesirable metabolites due to cellular senescence, for example, lipofuscin. In particular, the composition of the present invention can provide a synergistic effect for the rejuvenation of senescent cells or the delay or prevention of cellular senescence due to a combination of specific active ingredients. In addition, the composition of the present invention can effectively prevent, treat, or ameliorate cellular senescence-related diseases based on the above-described effects. In addition, the composition of the present invention can effectively prevent or ameliorate skin aging, loss of skin elasticity, skin wrinkles, or skin keratinization based on the above-described effects.

### [Description of Drawings]

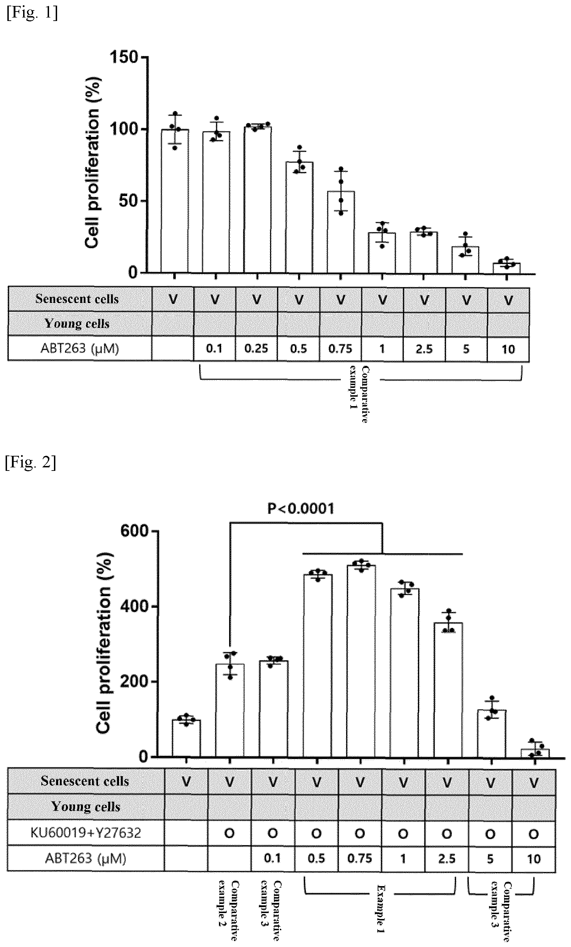

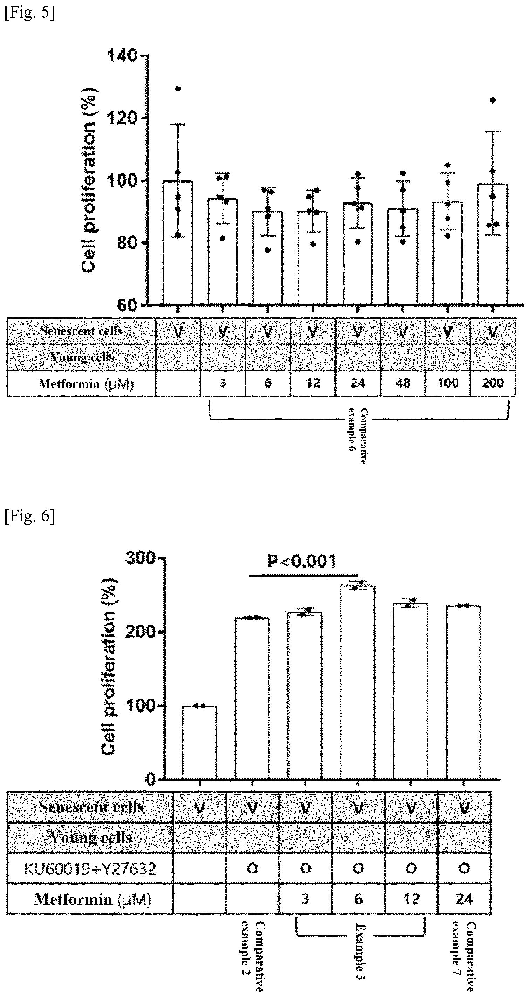

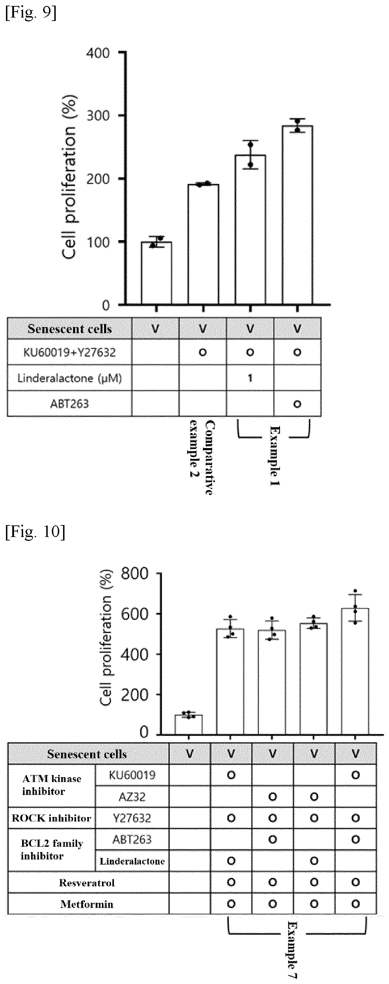

FIGS. 1 to 12 show the experimental results of comparing the cell division ability of senescent cells in cases where the cells were treated under the same conditions as one embodiment of the present invention (Examples 1 to 7) with other cases (Comparative Examples 1 to 7) using the Hoechst assay.

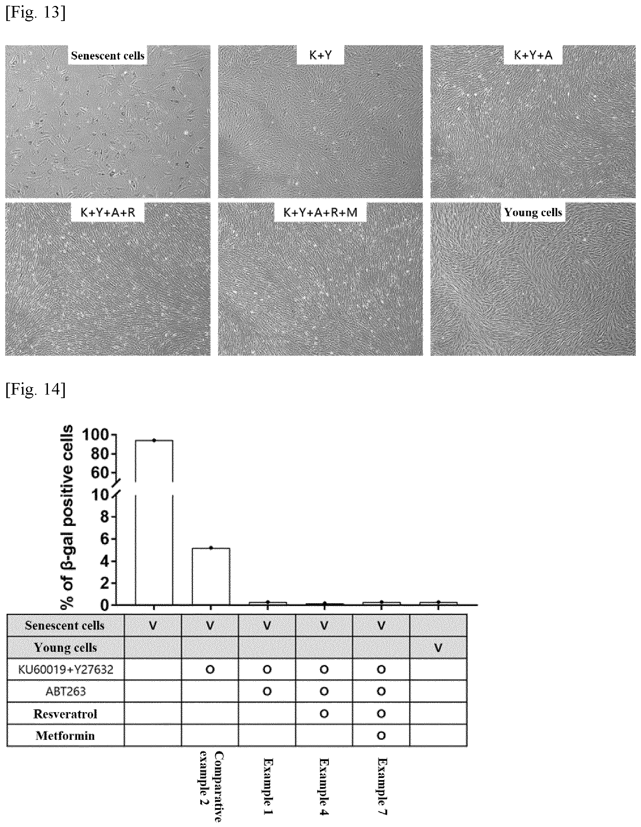

FIGS. 13 to 15 show the experimental results of comparing the degree of senescence of senescent cells in cases where the cells were treated under the same conditions as one embodiment of the present invention (Examples 1, 4, and 7) with another case (Comparative Example 2) using the beta-galactosidase staining (β-staining) method. K, KU60019; Y, Y27632; A, ABT263; R, Resveratrol; M, Metformin; K+Y, Comparative Example 2; K+Y+A, Example 1; K+Y+A+R, Example 4; and K+Y+A+R+M, Example 7.

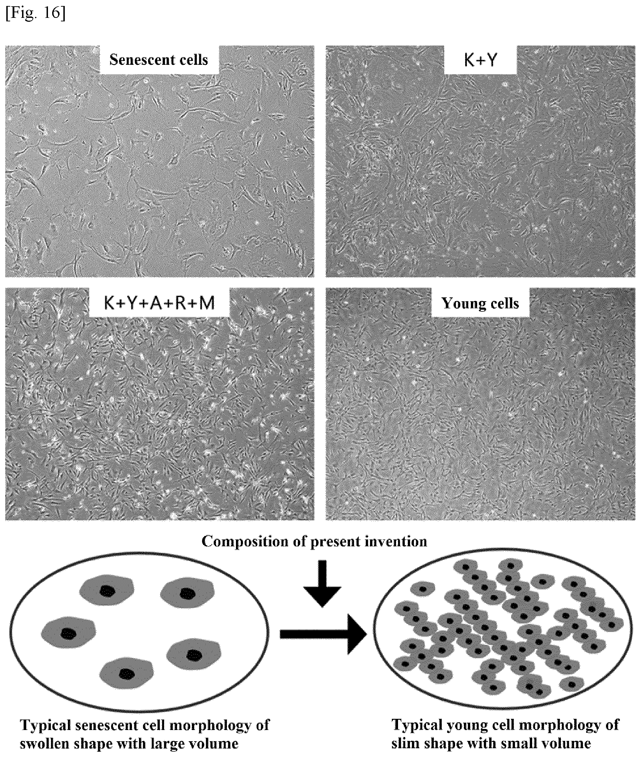

FIG. 16 shows the results of observing and comparing the morphology of senescent cells in cases where the cells were treated under the same conditions as one embodiment of the present invention (Examples 1, 4 and 7) with another case (Comparative Example 2). K, KU60019; Y, Y27632; A, ABT263; R, Resveratrol; M, Metformin; K+Y, Comparative Example 2; and K+Y+A+R+M, Example 7.

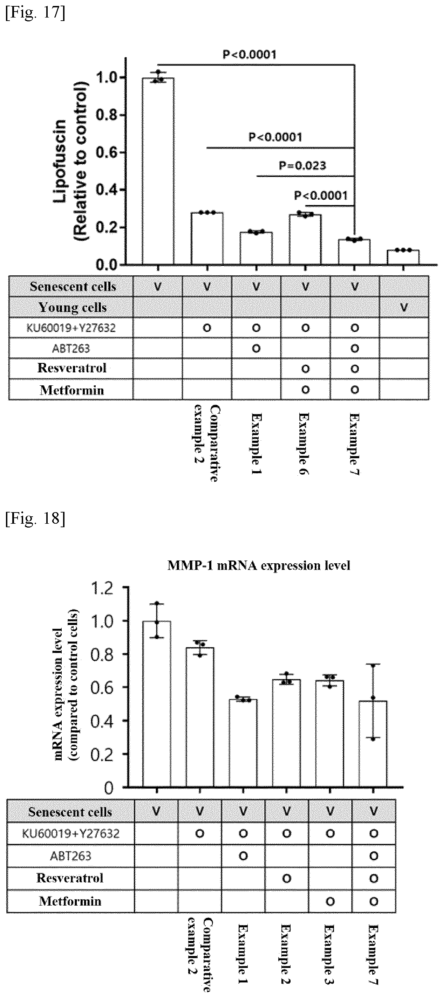

FIG. 17 shows the experimental results of comparing the amount of lipofuscin in senescent cells in cases where the cells were treated under the same conditions as one embodiment of the present invention (Examples 1, 6 and 7) with another case (Comparative Example 2).

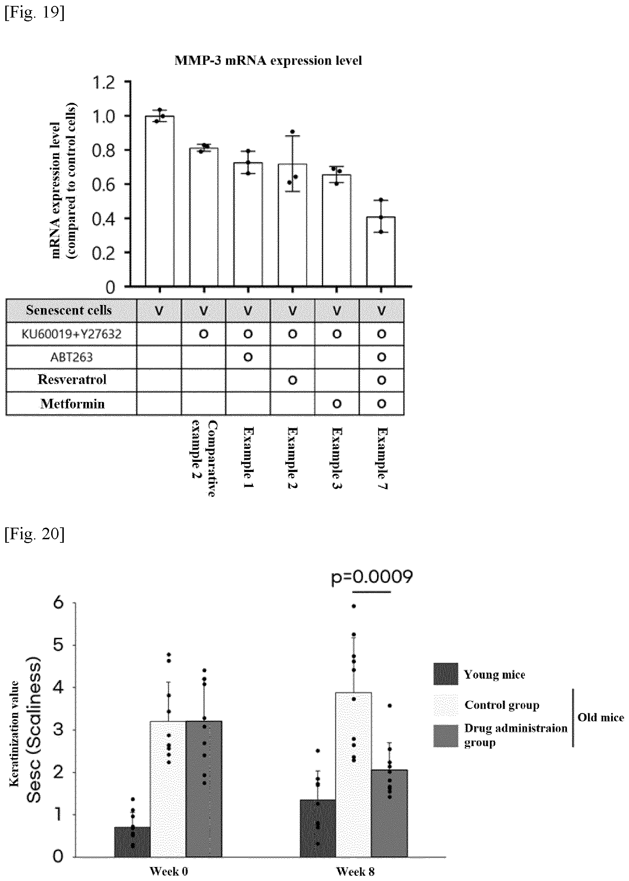

FIGS. 18 and 19 show the experimental results of comparing the mRNA expression levels of matrix metalloproteinase (MMP)-1 (FIG. 18) and MMP-3 (FIG. 19) in senescent cells in cases where the cells were treated under the same conditions as one embodiment of the present invention (Examples 1, 2, 3, and 7) with another case (Comparative Example 2).

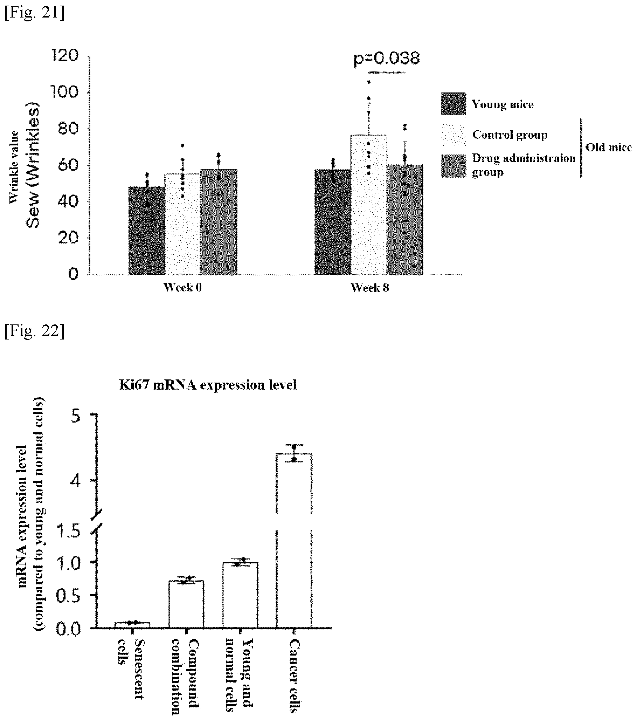

FIGS. 20 and 21 show the results of preclinical experiments on the effectiveness of ameliorating skin keratinization (FIG. 20) and wrinkles (FIG. 21) by the administration of the composition of the present invention.

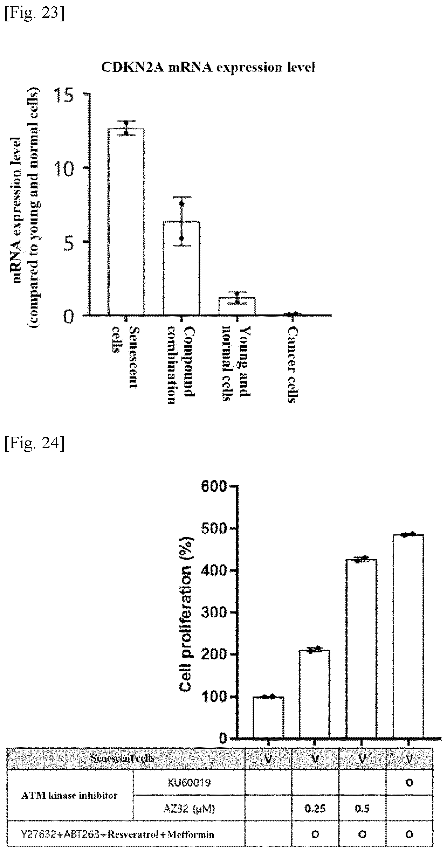

FIGS. 22 and 23 show the experimental results confirming the tumorigenicity of senescent cells in cases where the cells were treated under the same conditions as one embodiment of the present invention (Example 7; compound combination) by comparing the mRNA expression levels of Ki67 (FIG. 22) and CDKN2A (FIG. 23) with those of cancer cells.

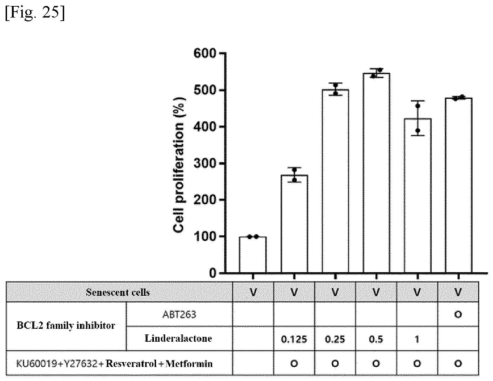

FIGS. 24 and 25 show the results of testing the cell division ability of senescent cells in cases where the cells were treated under conditions according to one embodiment of the present invention.

With regard to the circles in the tables included in the drawings, the circle in the "KU60019" item means that KU60019 was treated at a concentration of 0.5 µM, the circle in the "AZ32" item means that AZ32 was treated at a concentration of 0.5 µM, the circle in the "Y27632" item means that Y27632 was treated at a concentration of 2.5 µM, the circle in the "ABT263" item means that ABT263 was treated at a concentration of 0.75 µM, the circle in the "ABT737" item means that ABT737 was treated at a concentration of 0.75 µM, the circle in the "linderalactone" item means that linderalactone was treated at a concentration of 0.5 µM, and the circle in the "resveratrol" item means that resveratrol was treated at a concentration of 1.5 µM, and the circle in the "metformin" item means that metformin was treated at a concentration of 6 µM.

### [Modes of the Invention]

A composition for rejuvenating senescent cells or delaying or preventing cellular senescence of the present invention includes an ATM kinase inhibitor and a ROCK inhibitor, and one or more selected from the group consisting of a Bcl-2 inhibitor, a SIRT1 activator, and an AMPK activator.

In the present invention, an ATM kinase inhibitor refers to a substance that inhibits the action of ATM kinase, for example, the Ser15 phosphorylation action of p53. In one embodiment, the ATM kinase inhibitor is any compound known to inhibit ATM kinase. In a specific embodiment, the ATM kinase inhibitor is 'KU60019' (CAS 925701-46-8) (Chemical Formula 1) or 'AZ32' (CAS 2288709-96-4) (Chemical Formula 2) or a derivative or analog thereof.

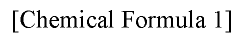

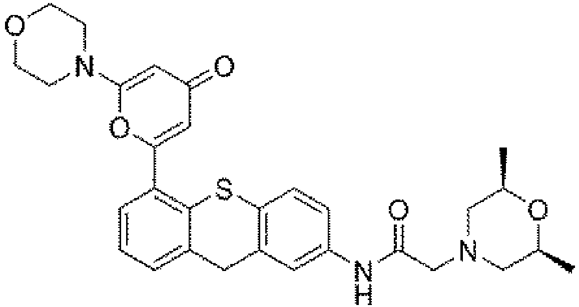

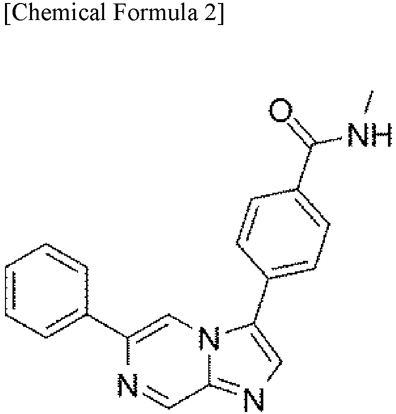

In the present invention, a ROCK inhibitor refers to a substance that inhibits the action of ROCK (ROCK1 and/or ROCK2), for example, the binding action of ROCK and Rho GTPase and/or the action mediating the activity of Rho GTPase. In one embodiment, the ROCK inhibitor is any compound known to inhibit ROCK. In a specific embodiment, the ROCK inhibitor is 'Y27632' (CAS 129830-38-2) (Chemical Formula 3) or a derivative or analog thereof.

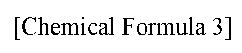

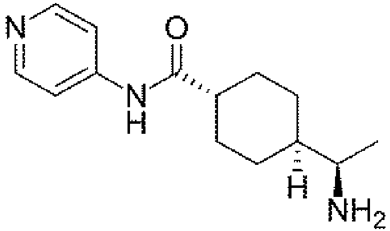

In the present invention, a Bcl-2 inhibitor refers to a substance that inhibits the action of Bcl-2, for example, binding to Bax and Bak proteins, which are apoptotic effectors. In one embodiment, the Bcl-2 inhibitor is any compound known to inhibit Bcl-2. In a specific embodiment, the Bcl-2 inhibitor is 'ABT263' (CAS 923564-51-6) (Chemical Formula 4), 'ABT737 (CAS 852808-04-9) (Chemical Formula 5), or 'linderalactone' (CAS 728-61-0) (Chemical Formula 6) or a derivative or analog thereof.

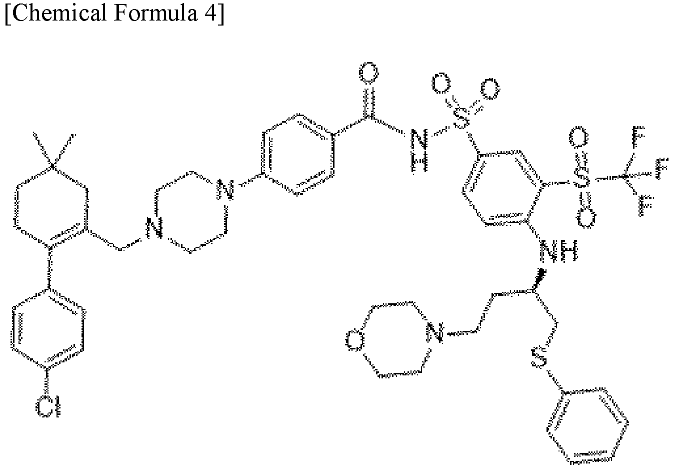

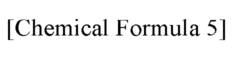

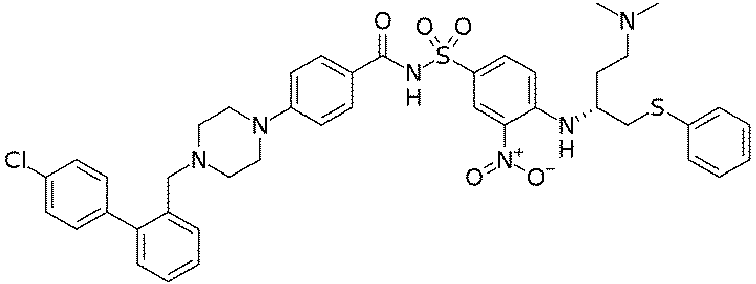

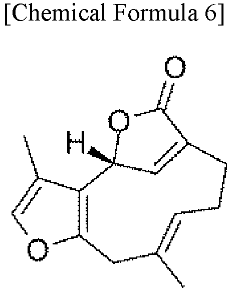

In the present invention, a SIRT1 activator refers to a substance that activates the action of SIRT1. In one embodiment, the SIRT1 activator is any compound known to activate SIRT1. In a specific embodiment, the SIRT1 activator is resveratrol or a derivative or analog thereof.

In the present invention, an AMPK activator refers to a substance that activates the action of AMPK. In one embodiment, the AMPK activator is any compound known to activate AMPK. In one embodiment, the AMPK activator is a mitochondrial complex 1 (MC1) inhibitor. In one embodiment, the MC1 inhibitor is any compound known to inhibit MC1. In a specific embodiment, the AMPK activator is metformin, which is also an MC1 inhibitor, or a derivative or analog thereof.

Unless otherwise specified, the compounds mentioned herein are intended to include salt forms of the compounds, for example, pharmaceutically, sitologically, and/or cosmetically acceptable salt forms.

In the present specification, rejuvenation of senescent cells means reverting various characteristics of senescent cells to be the same as or similar to those of young cells. For example, it may refer to reverse senescence in which old cells that have reached the Hayflick limit and no longer proliferate resume active proliferation like young cells, and/or reverse senescence in which senescence markers such as beta-galactosidase staining or lipofuscin are restored to the levels of young cells (e.g., to the expression level observed in young cells). In one embodiment, the composition of the present invention has at least one activity selected from the group consisting of cell proliferation increasing activity, lipofuscin accumulation decreasing activity, and beta-galactosidase activity decreasing activity.

Rejuvenation of senescent cells may be distinguished from tumorigenesis or carcinogenesis. For example, rejuvenation of senescent cells may be distinguished from tumorigenesis or carcinogenesis because the expression of cell proliferation factors (e.g., Ki67) and/or cell cycle regulators (e.g., CDKN2A) is regulated within a normal range (i.e., to expression levels observed in normal cells rather than levels observed in tumor or cancer cells).

The composition of the present invention may rejuvenate senescent cells through a reverse senescence effect that reverts senescent cells to a young cell state, such as by causing senescent cells that have reached the Hayflick limit to resume cell division or by reducing the accumulation of undesirable metabolites due to cellular senescence, such as lipofuscin. In this context, the composition of the present invention may be a composition for inducing reverse senescence.

In one embodiment of the present invention, the cell is a cell of an animal, for example, a mammal, for example, a human, and in a particular embodiment, it is a skin cell, for example, a skin fibroblast. In another particular embodiment, it is a colon cell, for example, a colon fibroblast.

In one embodiment, the composition of the present invention includes an ATM kinase inhibitor, a ROCK inhibitor, and a Bcl-2 inhibitor. In another embodiment, the composition of the present invention includes an ATM kinase inhibitor, a ROCK inhibitor, and a SIRT1 activator. In still another embodiment, the composition of the present invention includes an ATM kinase inhibitor, a ROCK inhibitor, and an AMPK activator.

In a preferred embodiment, the composition of the present invention includes an ATM kinase inhibitor, a ROCK inhibitor, and a Bcl-2 inhibitor, and includes one or more selected from the group consisting of a SIRT1 activator and an AMPK activator.

In a more preferred embodiment, the composition of the present invention includes all of an ATM kinase inhibitor, a ROCK inhibitor, a Bcl-2 inhibitor, a SIRT1 activator, and an AMPK activator.

In one embodiment, the composition of the present invention includes only the indicated active ingredients, namely, an ATM kinase inhibitor, a ROCK inhibitor, a Bcl-2 inhibitor (when included), a SIRT1 activator (when included), and an AMPK activator (when included). In another embodiment, the composition of the present invention also includes another active ingredient in addition to the indicated ingredients.

For higher expected effects of the present invention, the molar ratio of the ATM kinase inhibitor and the ROCK inhibitor of the composition of the present invention is 1:2.5 to 20, more preferably 1:2.5 to 15, more preferably 1:3 to 10, more preferably 1:3.5 to 8, more preferably 1:4 to 7, and more preferably 1:4.5 to 6.

In an embodiment including a Bcl-2 inhibitor, for higher expected effects of the present invention, the molar ratio of the ATM kinase inhibitor and the Bcl-2 inhibitor of the composition of the present invention is 1:0.25 to 6, more preferably 1:0.5 to 5, more preferably 1:1 to 4, more preferably 1:1.1 to 3, more preferably 1:1.2 to 2.5, and more preferably 1:1.3 to 2.

In an embodiment including a SIRT1 activator, for higher expected effects of the present invention, the molar ratio of the ATM kinase inhibitor and the SIRT1 activator of the composition of the present invention is 1:1.5 to 6, more preferably 1:1.8 to 5.5, more preferably 1:2 to 5, more preferably 1:2.1 to 4.5, more preferably 1:2.3 to 4.3, more preferably 1:2.5 to 4.

In an embodiment including an AMPK activator, for higher expected effects of the present invention, the molar ratio of the ATM kinase inhibitor to the AMPK activator of the composition of the present invention is 1:6 to 24, more preferably 1:7 to 22, more preferably 1:8 to 20, more preferably 1:9 to 18, more preferably 1:9.5 to 16, and more preferably 1:10 to 15.

The composition of the present invention may be in the form of the indicated active ingredient dissolved or suspended in a medium. For example, it may be in the form of an aqueous solution.

In one embodiment, the composition of the present invention is a composition provided for use without separate dilution or addition of a separate substance. In another embodiment, the composition of the present invention is a composition provided for use by dilution or suspension in a suitable medium (e.g., a solvent, e.g., water).

In an embodiment of the composition provided for use without separate dilution or addition of a substance, for higher expected effects, the composition of the present invention includes the ATM kinase inhibitor at a concentration of 0.125 µM or higher, for example, a concentration of 0.125 to 1.5 µM, more preferably, a concentration of 0.25 µM or higher, for example, a concentration of 0.25 to 1 µM, and based on this concentration, other active ingredients are present at concentrations in the indicated ratio ranges.

In the case of an embodiment of the composition provided for use with dilution or suspension in a suitable medium, based on a concentration of, for example, 0.5 µM of an ATM kinase inhibitor, the composition may include the ATM kinase inhibitor at a concentration of, for example, 1.5-fold (the concentration of the ATM kinase inhibitor is 0.75 µM) or more, for example, 2-fold, 3-fold, 4-fold, 5-fold, 6-fold, 7-fold, 8-fold, 9-fold, or 10-fold.

In one embodiment, the composition of the present invention is a pharmaceutical composition for preventing or treating a cellular senescence-related disease.

In another embodiment, the composition of the present invention is a functional food composition for preventing or ameliorating a cellular senescence-related disease, preventing or ameliorating skin aging, preventing or improving loss of skin elasticity, preventing or ameliorating skin wrinkles, or preventing or ameliorating skin keratinization.

In still another embodiment, the composition of the present invention is a cosmetic composition for preventing or ameliorating a cellular senescence-related disease, preventing or ameliorating skin aging, preventing or improving loss of skin elasticity, preventing or ameliorating skin wrinkles, or preventing or ameliorating skin keratinization.

**In** yet another embodiment, the composition of the present invention is a functional feed composition for preventing or ameliorating a cellular senescence-related disease, preventing or ameliorating skin aging, preventing or improving loss of skin elasticity, preventing or ameliorating skin wrinkles, or preventing or ameliorating skin keratinization.

**In** the present invention, a cellular senescence-related disease may be a skin cellular senescence-related disease, for example, a disease selected from the group consisting of hair loss, grey hair, wrinkles, xeroderma, xerosis, keratinization, melasma, age spots, delay of wound healing, dermatitis, psoriasis, scleroderma, vitiligo, and systemic lupus erythematosus.

In the present invention, a cellular senescence-related disease may be a lipofuscin accumulation-related disease, for example, a disease selected from the group consisting of lipofuscinosis, macular degeneration, neuronal ceroid lipofuscinoses (NCL), Stargardt disease, neurofibrillary tangles, brown atrophy of the heart, acromegaly, denervation atrophy, lipid myopathy, and chronic obstructive pulmonary disease (COPD).

In addition, in the present invention, a cellular senescence-related disease may be a disease selected from the group consisting of premature aging (Hutchinson-Gilford progeria syndrome or Werner syndrome), hunchback, osteoporosis, osteoarthritis, rheumatoid arthritis, benign prostatic hyperplasia, presbycusis, rhinitis, immunosenescence, post-transplantation late and chronic solid organ rejection, multiple sclerosis, Sjogren syndrome, Hashimoto thyroiditis, Graves disease, polymyositis, sarcopenia, Addison disease, glomerulonephritis, pernicious anemia, asthma, cystic fibrosis, pulmonary fibrosis, inflammatory bowel disease, autoimmune diabetes, type 2 diabetes mellitus, diabetic retinopathy, hypertension, arrhythmia, angina, COPD, ischemic heart disease, ischemia-reperfusion injury, post-angioplasty restenosis, atherosclerosis, coronary artery disease, gastrointestinal allergies, cancer, frailty, arteriolar diseases and graft-versus-host disease, conjunctivitis, glaucoma, cataracts, presbyopia, stroke, dementia, Alzheimer's disease, Parkinson's disease, and amyotrophic lateral sclerosis (ALS), but is not limited thereto, and may also include diseases known to those skilled in the art as diseases caused by cellular senescence.

The composition of the present invention may be formulated into a suitable formulation using one or more pharmaceutically and/or sitologically acceptable carriers, additives, and the like, in addition to the active ingredients of the present invention.

The composition of the present invention may be administered orally or parenterally at the time of clinical administration. In the case of parenteral administration, it may be administered by intraperitoneal injection, intrarectal injection, subcutaneous injection, intravenous injection, intramuscular injection, intrauterine epidural injection, intracerebrovascular injection or intrathoracic injection, and may be used in the form of a general pharmaceutical preparation.

The composition of the present invention may be used alone or in combination with methods using surgery, radiotherapy, hormone therapy, chemotherapy, and biological response modifiers.

The dosage of the composition of the present invention varies depending on the administration subject's body weight, age, sex, health conditions, and diet, administration time, administration method, excretion rate, and disease severity, and may be, for example, about 0.0001 to 100 mg per day per 1 kg of the administration subject's body weight.

The food composition of the present invention may be a health functional food including, for example, processed meat products, fish products, tofu, jelly, porridge, noodles such as ramen, seasonings such as soy sauce, soybean paste, red pepper paste, and mixed paste, sauces, confectionery, processed dairy products such as fermented milk or cheese, pickled foods such as kimchi or pickled vegetables, and beverage products such as fruit beverage, vegetable beverage, soy milk, and fermented beverage.

The feed composition of the present invention may also be in various forms and may be a feed composition for livestock (e.g., sheep, pigs, cows, horses, goats), laboratory test animals such as mice, rabbits, rats, and guinea pigs, and companion animals such as dogs and cats.

In one embodiment, the composition of the present invention is a cosmetic composition for preventing or ameliorating skin aging, preventing or improving loss of skin elasticity, preventing or ameliorating skin wrinkles, or preventing or ameliorating skin keratinization.

The cosmetic composition of the present invention may be formulated into a cosmetic formulation using one or more cosmetically acceptable carriers, additives, and the like, in addition to the active ingredient of the present invention. At this time, the carriers and additives may also include pharmaceutically acceptable ones.

The cosmetic composition of the present invention may be formulated into various forms, such as a mist, a moisturizing cream, a nutritional lotion, a nutritional cream, a massage cream, a pack, and a nutritional essence.

In one embodiment, the composition of the present invention is a medium composition or a medium additive composition for cell culture.

The medium composition of the present invention may be in a form in which a conventional medium component suitable for the cells to be cultured is added to the active ingredient of the present invention. For example, in the case of an embodiment of a medium composition for culturing human skin fibroblasts, it may be in a form in which the active ingredient of the present invention is added to Dulbecco's modified Eagle's medium (DMEM) at the indicated ratio.

In addition, the present invention relates to a method of rejuvenating senescent cells, including treating senescent cells with one or more selected from the group consisting of a Bcl-2 inhibitor, a SIRT1 activator, and an AMPK activator together with an ATM kinase inhibitor and a ROCK inhibitor.

In addition, the present invention relates to a method of preventing, treating or ameliorating a cellular senescence-related disease, including administering to a subject in need thereof one or more selected from the group consisting of a Bcl-2 inhibitor, a SIRT1 activator, and an AMPK activator together with an ATM kinase inhibitor and a ROCK inhibitor.

In addition, the present invention relates to a method of preventing or ameliorating skin aging, preventing or improving loss of skin elasticity, preventing or ameliorating skin wrinkles, or preventing or ameliorating skin keratinization, wherein the method of preventing or ameliorating skin aging, preventing or improving loss of skin elasticity, preventing or ameliorating skin wrinkles, or preventing or ameliorating skin keratinization includes administering to a subject in need thereof one or more selected from the group consisting of a Bcl-2 inhibitor, a SIRT1 activator, and an AMPK activator together with an ATM kinase inhibitor and a ROCK inhibitor.

In one embodiment, the method of the present invention treats or administers an ATM kinase inhibitor, a ROCK inhibitor, and a Bcl-2 inhibitor. In another embodiment, the method of the present invention treats or administers an ATM kinase inhibitor, a ROCK inhibitor, and a SIRT1 activator. In still another embodiment, the method of the present invention treats or administers an ATM kinase inhibitor, a ROCK inhibitor, and an AMPK activator.

In a preferred embodiment, the method of the present invention includes treating or administering one or more selected from the group consisting of a SIRT1 activator and an AMPK activator together with an ATM kinase inhibitor, a ROCK inhibitor, and a Bcl-2 inhibitor.

In a more preferred embodiment, the method of the present invention treats or administers all of an ATM kinase inhibitor, a ROCK inhibitor, a Bcl-2 inhibitor, a SIRT1 activator, and an AMPK activator.

For higher expected effects of the present invention, the molar ratio of the ATM kinase inhibitor and the ROCK inhibitor of the method of the present invention is 1:2.5 to 20, more preferably 1:2.5 to 15, more preferably 1:3 to 10, more preferably 1:3.5 to 8, more preferably 1:4 to 7, and more preferably 1:4.5 to 6.

In an embodiment of treating or administering a Bcl-2 inhibitor, for higher expected effects of the present invention, the molar ratio of the ATM kinase inhibitor and the Bcl-2 inhibitor of the method of the present invention is 1:0.25 to 6, more preferably 1:0.5 to 5, more preferably 1:1 to 4, more preferably 1:1.1 to 3, more preferably 1:1.2 to 2.5, and more preferably 1:1.3 to 2.

In an embodiment of treating or administering a SIRT1 activator, for higher expected effects of the present invention, the molar ratio of the ATM kinase inhibitor and the SIRT1 activator of the method of the present invention is 1:1.5 to 6, more preferably 1:1.8 to 5.5, more preferably 1:2 to 5, more preferably 1:2.1 to 4.5, more preferably 1:2.3 to 4.3, and more preferably 1:2.5 to 4.

In an embodiment of treating or administering an AMPK activator, for higher expected effects of the present invention, the molar ratio of the ATM kinase inhibitor and the AMPK activator of the method of the present invention is 1:6 to 24, more preferably 1:7 to 22, more preferably 1:8 to 20, more preferably 1:9 to 18, more preferably 1:9.5 to 16, and more preferably 1:10 to 15.

In the method of the present invention, the subject may be a mammal, for example, a human, a primate, and a livestock animal (e.g., a sheep, a pig, a cow, a horse, and a goat), a laboratory test animal, such as a mouse, a rabbit, a rat, and a guinea pig, and a companion animal, such as a dog or a cat.

In the method of the present invention, the administration may be oral administration or parenteral administration. Parenteral administration may be administration via intraperitoneal injection, intrarectal injection, subcutaneous injection, intravenous injection, intramuscular injection, intrauterine epidural injection, intracerebrovascular injection, or intrathoracic injection.

The method of the present invention may be used alone or in combination with methods using surgery, radiotherapy, hormone therapy, chemotherapy, and biological response modifiers.

In one embodiment, the method of the present invention is configured so that the ATM kinase inhibitor is administered at a single dose of 3 to 10 mg/kg, preferably 4 to 8 mg/kg, and more preferably 4.5 to 6 mg/kg.

Hereinafter, the present invention will be described in more detail through examples. These examples are only intended to illustrate the present invention, and therefore, the scope of the present invention is not to be construed as being limited by these examples.

### [Examples]

### Cell culture

The human dermal fibroblasts used in the experiment were samples collected from newborn skin (PCS-201-010; ATCC), and the cells were cultured in a culture solution of DMEM [25 mM glucose, 10% FBS (S001-01; Welgene), 100 units/ml penicillin, 10 µg/ml streptomycin, and 25 ng/ ml amphotericin B (LS203-01; Welgene)] under the conditions of 37 °C and 5% CO₂. Subculture was performed each time the cell confluency (cell distribution rate on the bottom surface of the cell culture vessel) did not exceed 80%, and the subculture continued from the first passage collected from the newborn skin to the 48th passage by newly culturing 1/4 of the cells. After the 48th passage, 1/2 of the cells were cultured. When the population doubling time (DT) exceeded 14 days and the senescence-associated β-galactosidase (SA-β) ratio exceeded 95%, experiments were conducted by considering the cells as sufficiently aged. On the other hand, experiments were conducted by considering only the cells that had been subcultured less than 10 times and had a population DT of one day or less as young cells.

The human breast skin fibroblast cells (CCD-986Sk) used in the experiment were samples collected from the breast of a 22-year-old black woman (CRL-1947; ATCC). The cells were cultured in a culture solution of DMEM [25 mM glucose, 10% FBS (S001-01; Welgene), 100 units/ml penicillin, 10 µg/ml streptomycin, and 25 ng/ml amphotericin B (LS203-01; Welgene)] under the conditions of 37 °C and 5% CO₂. Subculture was performed each time the cell confluency (cell distribution rate on the bottom surface of the cell culture vessel) did not exceed 80%, and the subculture continued from the initially taken first passage to the 15th passage by newly culturing 1/4 of the cells. After the 15th passage, 1/2 of the cells were cultured. The experiment was performed with the 19th passage cells. When the population DT exceeded 14 days and the SA-β ratio exceeded 95%, experiments were conducted by considering the cells as sufficiently aged.

The human colon fibroblast cells (CCD-18Co) used in the experiment were samples collected from the colon of a 2.5-month-old black female child (CRL-1459; ATCC). The cells were cultured in a culture solution of DMEM [25 mM glucose, 10% FBS (S001-01; Welgene), 100 units/ml penicillin, 10 µg/ml streptomycin, and 25 ng/ml amphotericin B (LS203-01; Welgene)] under the conditions of 37 °C and 5% CO₂. Subculture was performed each time the cell confluency (cell distribution rate on the bottom surface of the cell culture vessel) did not exceed 80%, and the subculture continued from the initially taken first passage to the 10th passage by newly culturing 1/4 of the cells. After the 10th passage, 1/2 of the cells were cultured. The experiment was performed with the 16th passage cells. When the population DT exceeded 14 days and the SA-β ratio exceeded 95%, experiments were conducted by considering the cells as sufficiently aged.

### Example 1

Senescent cells were prepared and treated with a medium containing the ATM kinase inhibitor (KU60019 or AZ32) at a concentration of 0.5 µM, the ROCK inhibitor (Y27632) at a concentration of 2.5 µM, and the Bcl-2 inhibitor (ABT263, ABT737, or linderalactone) at a concentration of 0.5 to 2.5 µM, once every 4 days for 16 days for a total of 4 times.

### Comparative Example 1

The same experiment as Example 1 was performed, except that the ATM kinase inhibitor and the ROCK inhibitor were not added, and only the Bcl-2 inhibitor was added to the medium at a concentration of 0.1 to 10 µM.

### Comparative Example 2

The same experiment as Example 1 was performed, except that the Bcl-2 inhibitor was not added and only the ATM kinase inhibitor and the ROCK inhibitor were added to the medium.

### Comparative Example 3

The same experiment as Example 1 was performed, except that the Bcl-2 inhibitor was added to the medium at a concentration of 0.1 µM or 5 to 10 µM.

### Example 2

The same experiment as Example 1 was performed, except that instead of the Bcl-2 inhibitor, a SIRT1 activator (resveratrol) was added to the medium at a concentration of 1 to 2 µM.

### Comparative Example 4

The same experiment as Example 2 was performed, except that the ATM kinase inhibitor and the ROCK inhibitor were not added, and only the SIRT1 activator was added to the medium at concentrations of 0.1 to 50 µM.

### Comparative Example 5

The same experiment as Example 2 was performed, except that the SIRT1 activator was added to the medium at concentrations of 4 to 10 µM.

### Example 3

The same experiment as Example 1 was performed, except that instead of the Bcl-2 inhibitor, an AMPK activator (metformin) was added to the medium at a concentration of 3 to 12 µM.

### Comparative Example 6

The same experiment as Example 3 was performed, except that the ATM kinase inhibitor and the ROCK inhibitor were not added, and only the AMPK activator was added to the medium at concentrations of 3 to 200 µM.

### Comparative Example 7

The same experiment as Example 3 was performed, except that the AMPK activator was added to the medium at a concentration of 24 µM.

### Example 4

The same experiment as Example 1 was performed, except that the Bcl-2 inhibitor was added to the medium at concentrations of 0.5 to 0.75 µM, and the SIRT1 activator was further added to the medium at concentrations of 1.5 to 2 µM.

### Example 5

The same experiment as Example 4 was performed, except that instead of the SIRT1 activator, the AMPK activator was added to the medium at a concentration of 6 µM.

### Example 6

The same experiment as Example 2 was performed, except that the AMPK activator was further added to the medium at a concentration of 6 µM.

### Example 7

The same experiment as Example 4 was performed, except that the AMPK activator was further added to the medium at a concentration of 6 µM.

Examples 1 to 7 and Comparative Examples 1 to 7 are summarized in Table 1 below.

**[Table 1]**

| | Example 1 | Example 2 | Example 3 | Example 4 | Example 5 | Example 6 | Example 7 |
|---|---|---|---|---|---|---|---|
| ATM kinase inhibito r | 0.5 | 0.5 | 0.5 | 0.5 | 0.5 | 0.5 | 0.5 |
| ROCK inhibito r | 2.5 | 2.5 | 2.5 | 2.5 | 2.5 | 2.5 | 2.5 |
| Bcl-2 inhibito r | 0.5 to 2.5 | | | 0.5 to 0.75 | 0.5 to 0.75 | | 0.5 to 0.75 |
| SIRT1 activato r | | 1 to 2 | | 1.5 to 2 | | 1.5 to 2 | 1.5 to 2 |
| AMPK activato r | | | 3 to 12 | | 6 | 6 | 6 |
| | Comparati ve Example 1 | Comparati ve Example 2 | Comparati ve Example 3 | Comparati ve Example 4 | Comparati ve Example 5 | Comparati ve Example 6 | Comparati ve Example 7 |
| ATM kinase inhibito r | | 0.5 | 0.5 | | 0.5 | | 0.5 |
| ROCK inhibito r | | 2.5 | 2.5 | | 2.5 | | 2.5 |
| Bcl-2 inhibito r | 0.1 to 10 | | 0.1 or 5 to 10 | | | | |
| SIRT1 activato r | | | | 0.1 to 50 | 4 to 10 | | |
| AMPK activato r | | | | | | 3 to 200 | 24 |

| | | | | | | | |
|---|---|---|---|---|---|---|---|
| * Units, µM. | | | | | | | |

### Experimental Example 1. Experiment to confirm cell division

The cell division ability of the senescent cells treated with the compounds in the above examples and comparative examples was investigated.

The cells to be used in the experiment were transplanted into a plate three days before the experiment. The cells treated with the compounds were stained with 10 µg/ml Hoechst 33342 for 30 minutes and washed twice with phosphate-buffered saline (PBS), and the amount of the cells was quantified using a plate reader (FLUOstar Omega) at wavelengths of ex/em: 355/460 nm.

FIGS. 1 to 10 show the experimental results for each example and comparative example in which human skin fibroblasts collected from newborn skin were used as senescent cells.

Senescent cells that have reached the Hayflick limit by subculture no longer divide. However, as in previous studies, it was confirmed that when treated with an ATM kinase inhibitor and a ROCK inhibitor (Comparative Example 2), cell division resumed as though the cells had been rejuvenated and become young cells. In addition, when treated with specific concentrations of the Bcl-2 inhibitor, the SIRT1 activator, or the AMPK activator (Examples 1 to 3), the cell rejuvenation effect was found to be more prominent, and the effect was particularly good when the cells were treated with the Bcl-2 inhibitor (Example 1) (FIGS. 1 to 3).

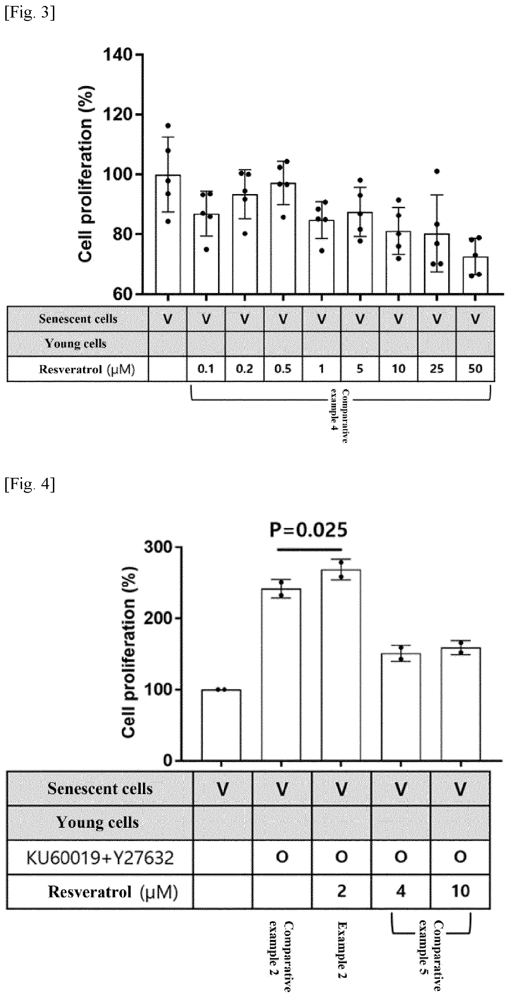

Meanwhile, when the Bcl-2 inhibitor, the SIRT1 activator, or the AMPK activator was treated alone (Comparative Examples 1, 4, and 6), rejuvenation of the senescent cells was not induced (FIGS. 4 to 6).

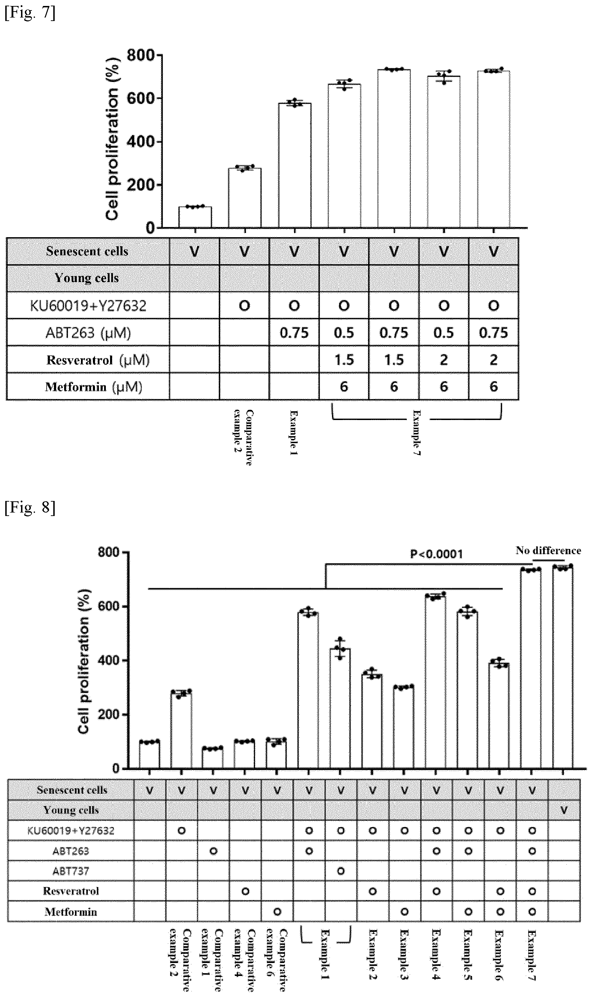

In addition, based on when the ATM kinase inhibitor and the ROCK inhibitor were included, the cell rejuvenation effect was more prominent when the SIRT1 activator or the AMPK activator was further treated together with the Bcl-2 inhibitor (Examples 4 and 5) than when only the Bcl-2 inhibitor was further treated (Example 1) (FIG. 7).

In addition, based on when the ATM kinase inhibitor and the ROCK inhibitor were included, the cell rejuvenation effect was more prominent when both the SIRT1 activator and the AMPK activator were treated together (Example 6) than when only one selected from the SIRT1 activator and the AMPK activator was further treated (Examples 2 and 3) (FIG. 7).

Based on when the ATM kinase inhibitor and the ROCK inhibitor were included, the cell rejuvenation effect was more prominent when two selected from among the Bcl-2 inhibitor, the SIRT1 activator, and the AMPK activator were combined (Examples 4 to 6), particularly when the Bcl-2 inhibitor was included (Examples 4 and 5) (FIG. 7).

In addition, when the cells were treated with all of the ATM kinase inhibitor, the ROCK inhibitor, the Bcl-2 inhibitor, the SIRT1 activator, and the AMPK activator (Example 7), the cell rejuvenation effect was better than in the above cases, and the senescent cells treated in this way exhibited characteristics that were not different from those of young cells (FIGS. 7 and 8).

In addition, when AZ32 was used instead of KU60019 as an ATM kinase inhibitor and when ABT737 or linderalactone was used instead of ABT263 as a Bcl-2 inhibitor, the results showed the same pattern (FIGS. 8 to 10). In particular, AZ32 and linderalactone, which are compounds with a molecular weight of 500 Da or less, have the advantage of being easily absorbed into the skin, which may be advantageous when applied to cosmetics.

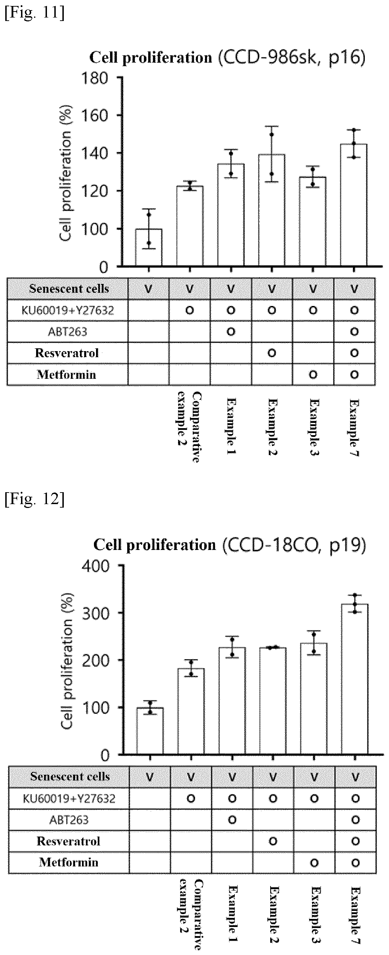

FIGS. 11 and 12 show the experimental results when other cells (human breast skin fibroblasts (CCD-986Sk) and human colon fibroblasts (CCD-18Co)) were used as senescent cells, respectively, and the results showed the same pattern as the previous results.

### Experimental Example 2. SA-β-gal staining experiment

To investigate the degree of aging of senescent cells treated with the compounds in the above examples and comparative examples, beta-galactosidase staining, a marker of cellular senescence, was performed.

SA-β-gal staining was performed with reference to the method of the X-gal-based Senescence β-Galactosidase Staining Kit (Cell Signaling Technology, #9860). Cells were transplanted three days before the experiment. The cells were washed with PBS, fixed with 4% formaldehyde for three minutes, and then washed twice with PBS. Then, the cells were stained with a β-gal solution (1 mg/ml X-gal, 5 mM K₄Fe(CN)₆, 5 mM K₃Fe(CN)₆, 2 mM MgCl₂, 150 mM NaCl, pH 6.0) in a 37 °C incubator for 24 hours. The next day, the cells were washed twice with PBS again, and the degree of cell staining was observed, and the cells were counted using an Olympus CKX53 microscope.

FIGS. 13 and 14 show the experimental results for each example and comparative example where human skin fibroblasts collected from newborn skin were used as senescent cells.

Unlike young cells, senescent cells are stained blue by beta-galactosidase staining, which is one of the biomarkers. It was confirmed that when these senescent cells were treated with the compounds, the number of unstained cells increased. In particular, it was found that, compared the case where the ATM kinase inhibitor and ROCK inhibitor were treated (Comparative Example 2), the effect was better when the ATM kinase inhibitor + ROCK inhibitor + Bcl-2 inhibitor were treated (Example 1), when the ATM kinase inhibitor + ROCK inhibitor + Bcl-2 inhibitor + SIRT1 activator were treated (Example 4), and when the ATM kinase inhibitor + ROCK inhibitor + Bcl-2 inhibitor + SIRT1 activator + AMPK activator were treated (Example 7).

FIG. 15 shows the experimental results when other cells (human colonic fibroblasts (CCD-18Co)) were used as senescent cells, and the results showed the same pattern as the previous results.

### Experimental Example 3. Observation of cell morphology

In above Experimental Example 2, the senescent cells of each example and comparative example before staining were observed under a microscope.

FIG. 16 shows the experimental results for each example and comparative example when human skin fibroblasts collected from newborn skin were used as senescent cells.

Senescent cells have a swollen shape with a large volume, whereas young cells have a slim and small volume shape in comparison. It was shown that when the senescent cells were treated with the compounds, they were shown to change into the typical slim and small-volume shape of young cells. In particular, it was found that the effect was better when the ATM kinase inhibitor + ROCK inhibitor + Bcl-2 inhibitor + SIRT1 activator + AMPK activator were treated (K+Y+A+R+M, Example 7) compared to when only the ATM kinase inhibitor and the ROCK inhibitor were treated (K+Y, Comparative Example 2).

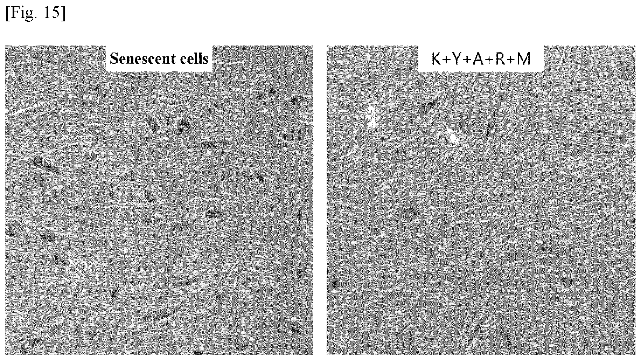

This pattern may also be confirmed in the SA-β staining experiment results of Experimental Example 2 (FIGS. 13 and 15).

### Experimental Example 4. Lipofuscin quantification experiment

The lipofuscin in senescent cells treated with the compounds in the above examples and comparative examples was quantified.

The amount of lipofuscin per cell was quantified using a FACSCanto II flow cytometer. Taking advantage of the fact that lipofuscin is autofluorescent, the values were measured without staining at a wavelength of 488 nm with a 530/30 nm bandpass filter (fluorescein isothiocyanate (FITC) channel).

FIG. 17 shows the experimental results for each example and comparative example when human skin fibroblasts collected from newborn skin were used as senescent cells.

In senescent cells, lipofuscin, a type of metabolic waste that is produced when various metabolites are digested or processed in lysosomes within cells, is produced. Lipofuscin is a yellowish-brown pigment granule, and it is not produced in young cells where metabolic waste processing is actively carried out, but when produced in old cells, it causes various degenerative diseases.

It was confirmed that when the senescent cells were treated with the compounds, lipofuscin was reduced. In particular, it was shown that, compared to the case where the ATM kinase inhibitor and the ROCK inhibitor were treated (Comparative Example 2), the effect was better when the Bcl-2 inhibitor was further treated (Example 1) and when the SIRT1 activator and the AMPK activator were additionally treated thereto (Example 7).

### Experimental Example 5. Experiment to confirm expression levels of MMP1 and MMP3

The expression levels of MMP1 and MMP3 in senescent cells treated with the compounds in the above examples and comparative examples were confirmed by quantitative polymerase chain reaction (q-PCR). MMP1 and MMP3 are enzymes that decompose the extracellular matrix and are highly related to skin elasticity and skin wrinkles.

Specifically, 10 µl of reagent (TOPreal qPCR 2X PreMIX), 1 µl of primer 1 (5 to 10 pmol/µl), 1 µl of primer 2 (5 to10 pmol/µl), 1 µl of template RNA, and distilled water (RNase-free) were added to a microtube to make a total of 20 µl, and the mixture was allowed to react at 95 °C for 10 minutes, followed by 35 additional PCR cycles (95 °C for 10 seconds, 60 °C for 15 seconds, and 72 °C for 15 seconds).

The primers for MMP1 and MMP3 used at this time are as shown in Table 2 below.

**[Table 2]**

| | Direction | Primer sequence (5'→3') |
|---|---|---|
| MMP1 | Forward | GGCCACAAAGTTGATGCAGT |
| | Reverse | TGCTACGGCAATGAAATGGAG |
| MMP3 | Forward | ACTGTGTTGGCCATAGAGCAC |
| | Reverse | GGGAACTGGCCCTAAGAAACA |

FIGS. 18 and 19 show the experimental results for each example and comparative example when human skin fibroblasts collected from newborn skin were used as senescent cells. It was found that when the senescent cells were treated with the compounds, the mRNA expression levels of MMP1 and MMP3 were decreased. In particular, it was found that, compared to the case where only the ATM kinase inhibitor and the ROCK inhibitor were treated (Comparative Example 2), the effect of decreasing the mRNA expression of MMP1 and MMP3 was better when the ATM kinase inhibitor and the ROCK inhibitor were treated together with the Bcl-2 inhibitor (Example 1), the SIRT1 activator (Example 2), or the AMPK activator (Example 3) and when all of the ATM kinase inhibitor + ROCK inhibitor + Bcl-2 inhibitor + SIRT1 activator + AMPK activator were treated together (Example 7).

### Experimental Example 6. Preclinical experiment on the efficacy of ameliorating skin keratinization and wrinkles

A preclinical experiment was conducted with dilute brown agouti (DBA) mice (species and strain: DBA/1J, producer and purchaser: Hana Corporation) to determine the efficacy of ameliorating skin keratinization and wrinkles.

DBA mice are known to be suitable for aging characteristic analysis and are widely used in metabolic marker research through bioimaging analysis, so they were selected to confirm the degree of aging using analytical equipment. The mice were all male, and ten 5-week-old mice were prepared as young mice, and twenty 16-month-old mice were prepared as old mice. Upon acquisition, the animals were inspected for appearance, and after a seven-day acclimatization period, they were used for animal experiments. During the experimental period, when separating groups, the animals were individually marked on the tails with a permanent marker, and individual identification cards were attached to the breeding boxes. When separating groups, the old mice experimental group was randomly separated into two groups, with 10 mice per group, the day before the beginning of the experiment.

An ATM kinase inhibitor (KU60019) 4.93 mg/kg, a ROCK inhibitor (Y27632) 11.13 mg/kg, a Bcl-2 inhibitor (ABT263) 13.16 mg/kg, a SIRT1 activator (resveratrol) 6.16 mg/kg, and an AMPK activator (metformin) 13.95 mg/kg were intravenously administered each time, twice a week for a total of 8 weeks (drug administration group).

When photographing the skin, the animal was anesthetized by inhaling 3% isoflurane gas. Once the animal was anesthetized and motionless, it was moved to a Skinscope device (Visioscan VC 20plus) and photographed, and the keratinization value and the wrinkle value were obtained simultaneously.

The results are as shown in FIGS. 20 and 21.

In the case of the old mice, the keratinization value and the wrinkle value were higher than in young mice, but it was shown that that the keratinization value and the wrinkle value were reduced by the drug administration to levels almost similar to those of young mice.

### Experimental Example 7. Experiment to confirm tumorigenicity

To determine whether the cell rejuvenation effect was due to carcinogenesis or tumorigenesis of the cells, the expression levels of Ki67, known as a cell proliferation factor, and CDKN2A, known as a cell cycle regulator, were compared with those of cancer cells.

The same q-PCR method as in Experimental Example 5 was used, except that the primers in Table 3 below were used.

**[Table 3]**

| | Direction | Primer sequence (5'→3') |
|---|---|---|
| MKI67 | Forward | GAGAGTAACGCGGAGTGTCA |
| | Reverse | GTTTGCCTTTGCAACCTTGG |
| CDKN2A | Forward | TCGAAGTTAAAGACGCGAAGT |
| | Reverse | TAACTGGAGCCGAAGTCTCCT |

The results are shown in FIGS. 22 and 23. Here, the "compound combination" item refers to a case in which the ATM kinase inhibitor (KU60019 treated at a concentration of 0.5 µM) + ROCK inhibitor (Y27632 treated at a concentration of 2.5 µM) + Bcl-2 inhibitor (ABT263 treated at a concentration of 0.75 µM) + SIRT1 activator (resveratrol treated at a concentration of 1.5 µM) + AMPK activator (metformin treated at a concentration of 6 µM) were treated together (Example 7). It was found that when the senescent cells were treated with the compounds, the expression of Ki67, known as a cell proliferation factor, was increased within a normal range, and the expression of CDKN2A, known as a cell cycle regulator, was also decreased within a normal range. This means that the combination of the substances according to the present invention may be safely used without concerns about tumorigenesis or carcinogenesis of cells.

### Experimental Example 8. Experiment on the effect of further concentration changes

An additional experiment was conducted using the same method as Experimental Example 1, but with a greater variety of concentrations of the treated substances.

Table 4 shows the experimental results when the ATM kinase inhibitor (KU60019) + ROCK inhibitor (Y27632) + Bcl-2 inhibitor (ABT263) + SIRT1 activator (resveratrol) + AMPK activator (metformin) were treated together, with the ATM kinase inhibitor treated at different concentrations of 0.125 to 1 µM, and the other substances (YARM) treated at different concentrations of 0.25 to 1X. Each result is expressed based on the cell division ability of the untreated group (100%). At this time, YARM 1X refers to the case in which 2.5 µM ROCK inhibitor, 0.75 µM Bcl-2 inhibitor, 1.5 µM SIRT1 activator, and 6 µM AMPK activator were treated.

It was shown that an excellent cell rejuvenation effect was achieved even when the concentration of the ATM kinase inhibitor was varied within the molar ratio of the present invention.

**[Table 4]**

| | KU60019 | | | |
|---|---|---|---|---|
| | 0.125µM | 0.25µM | 0.5µM | 1µM |
| YARM 0.25X | 242.5% | 206.2% | | |
| YARM 0.5X | | 277.4% | 297.3% | |
| YARM 1X | | 357.5% | 669.4% | 412.5% |

Table 5 shows the experimental results when the ATM kinase inhibitor (KU60019) + ROCK inhibitor (Y27632) + Bcl-2 inhibitor (ABT263) + SIRT1 activator (resveratrol) + AMPK activator (metformin) were treated together, with the ROCK inhibitor treated at different concentrations of 1.25 to 10 µM, and the other substances (KARM) treated at different concentrations of 0.25 to 1X. Each result is expressed based on the cell division ability of the untreated group (100%). At this time, KARM 1X refers to the case in which 0.5 µM ATM kinase inhibitor, 0.75 µM Bcl-2 inhibitor, 1.5 µM SIRT1 activator, and 6 µM AMPK activator were treated.

It was shown that an excellent cell rejuvenation effect was achieved even when the concentration of the ROCK inhibitor was varied within the molar ratio of the present invention.

**[Table 5]**

| | Y27632 | | | | |
|---|---|---|---|---|---|
| | 1.25µM | 2.5µM | 5µM | 7.5µM | 10µM |
| KARM 0.25X | 176.9% | 271.9% | | | |
| KARM 0.5X | 289.6% | 548.0% | 361.1% | | |
| KARM 1X | 576.1% | 597.4% | 419.3% | 427.5% | 365.2% |

FIG. 24 shows the experimental results when the ATM kinase inhibitor + ROCK inhibitor + Bcl-2 inhibitor + SIRT1 activator + AMPK activator were treated together, but AZ32 was used as an ATM kinase inhibitor.

It was shown that an excellent cell rejuvenation effect was achieved even when AZ32 was used as an ATM kinase inhibitor.

FIG. 25 shows the experimental results when the ATM kinase inhibitor + ROCK inhibitor + Bcl-2 inhibitor + SIRT1 activator + AMPK activator were treated together, but linderalactone was used as a Bcl-2 inhibitor.

It was shown that an excellent cell rejuvenation effect was achieved even when linderalactone was used as a Bcl-2 inhibitor.

## Claims

1. A composition for rejuvenating senescent cells or delaying or preventing cellular senescence, comprising an ataxia telangiectasia mutated (ATM) kinase inhibitor and a Rho-associated, coiled-coil containing protein kinase (ROCK) inhibitor, and one or more selected from the group consisting of a Bcl-2 inhibitor, a sirtuin 1 (SIRT1) activator, and an AMP-activated protein kinase (AMPK) activator.

2. The composition of claim 1, comprising a Bcl-2 inhibitor and one or more selected from the group consisting of a SIRT1 activator and an AMPK activator.

3. The composition of claim 1, comprising all of a Bcl-2 inhibitor, a SIRT1 activator, and an AMPK activator.

4. The composition of claim 1, wherein a molar ratio of the ATM kinase inhibitor and the ROCK inhibitor is 1:2.5 to 20;
a molar ratio of the ATM kinase inhibitor and the Bcl-2 inhibitor is 1:0.25 to 6;
a molar ratio of the ATM kinase inhibitor and the SIRT1 activator is 1:1.5 to 6; and
a molar ratio of the ATM kinase inhibitor and the AMPK activator is 1:6 to 24.

5. The composition of claim 1, wherein the composition has one or more activities selected from the group consisting of cell proliferation increasing activity, lipofuscin accumulation decreasing activity, and beta-galactosidase activity decreasing activity.

6. The composition of claim 1, wherein the composition is a pharmaceutical composition for preventing or treating a cellular senescence-related disease.

7. The composition of claim 1, wherein the composition is a functional food composition for preventing or ameliorating a cellular senescence-related disease, preventing or ameliorating skin aging, preventing or improving loss of skin elasticity, preventing or ameliorating skin wrinkles, or preventing or ameliorating skin keratinization.

8. The composition of claim 1, wherein the composition is a cosmetic composition for preventing or ameliorating a cellular senescence-related disease, preventing or ameliorating skin aging, preventing or improving loss of skin elasticity, preventing or ameliorating skin wrinkles, or preventing or ameliorating skin keratinization.

9. The composition of claim 1, wherein the composition is a functional feed composition for preventing or ameliorating a cellular senescence-related disease, preventing or ameliorating skin aging, preventing or improving loss of skin elasticity, preventing or ameliorating skin wrinkles, or preventing or ameliorating skin keratinization.

10. The composition of claim 6, wherein the composition is a pharmaceutical composition for preventing or treating a skin cellular senescence-related disease.

11. The composition of claim 10, wherein the composition is a pharmaceutical composition for preventing or treating a disease selected from the group consisting of hair loss, grey hair, wrinkles, xeroderma, xerosis, keratinization, melasma, age spots, delay of wound healing, dermatitis, psoriasis, scleroderma, vitiligo, and systemic lupus erythematosus.

12. The composition of claim 6, wherein the composition is a pharmaceutical composition for preventing or treating a lipofuscin accumulation-related disease.

13. The composition of claim 12, wherein the composition is a pharmaceutical composition for preventing or treating a disease selected from the group consisting of lipofuscinosis, macular degeneration, neuronal ceroid lipofuscinoses (NCL), Stargardt disease, neurofibrillary tangles, brown atrophy of the heart, acromegaly, denervation atrophy, lipid myopathy, and chronic obstructive pulmonary disease (COPD).

14. The composition of claim 6, wherein the composition is a pharmaceutical composition for preventing or treating a disease selected from the group consisting of premature aging (Hutchinson-Gilford progeria syndrome or Werner syndrome), hunchback, osteoporosis, osteoarthritis, rheumatoid arthritis, benign prostatic hyperplasia, presbycusis, rhinitis, immunosenescence, post-transplantation late and chronic solid organ rejection, multiple sclerosis, Sjogren syndrome, Hashimoto thyroiditis, Graves disease, polymyositis, sarcopenia, Addison disease, glomerulonephritis, pernicious anemia, asthma, cystic fibrosis, pulmonary fibrosis, inflammatory bowel disease, autoimmune diabetes, type 2 diabetes mellitus, diabetic retinopathy, hypertension, arrhythmia, angina, COPD, ischemic heart disease, ischemia-reperfusion injury, post-angioplasty restenosis, atherosclerosis, coronary artery disease, gastrointestinal allergies, cancer, frailty, arteriolar diseases and graft-versus-host disease, conjunctivitis, glaucoma, cataracts, presbyopia, stroke, dementia, Alzheimer's disease, Parkinson's disease, and amyotrophic lateral sclerosis (ALS).

15. The composition of claim 1, wherein the ATM kinase inhibitor is KU60019 or AZ32;
the ROCK inhibitor is Y27632; the Bcl-2 inhibitor is ABT263, ABT737 or linderalactone;
the SIRT1 activator is resveratrol; and
the AMPK activator is metformin.
